# EUROPÄISCHE PATENTSCHRIFT

(11) **EP 0 400 522 B1**
(45) Veröffentlichungstag und Bekanntmachung des Hinweises auf die Patenterteilung: **04.01.1995**
(21) Anmeldenummer: 90110055.2
(22) Anmeldetag: 28.05.1990
(51) Int. Cl.: A61K 9/14, A61K 9/50, A61K 9/58, A61K 9/16

(54) **Verfahren zur Herstellung mikronisierter bioabbaubarer Partikel**
Process for the preparation of micronised biodegradable particles
Procédé pour la préparation des particules micronisées biodégradables

(30) Priorität: 31.05.1989 DE 3917617
(43) Veröffentlichungstag der Anmeldung: 05.12.1990
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Zierenberg, Bernd, Dr.-Dipl.-Chem., D-6530 Bingen (DE); Muacevic, Gojko, Dr., D-6507 Ingelheim am Rhein (DE)

(56) Entgegenhaltungen:
- EP-A- 0 269 921
- WO-A-87/05803
- WO-A-87/07502
- CH-A- 665 558
- US-A- 4 818 542
- PATENT ABSTRACTS OF JAPAN vol. 9, no. 180 (C-293)(1903) 25. Juli 1985

## Beschreibung

Die Erfindung betrifft ein Verfahren zur Herstellung mikronisierter bioabbaubarer Partikel.

Auf dem Gebiet der inhalativen Arzneiformen, nehmen Depotformen, die den Wirkstoff über einen längeren Zeitraum abgeben und auf der Lungenoberfläche freisetzen eine besondere Stellung ein.

So wäre zum Beispiel für einen Asthmatiker eine inhalative Depotform besonders vorteilhaft, die ihm einen medikamentösen Schutz über einen Zeitraum von ca. 12 bis 24 Stunden gewährleistet. Um diesen Zweck zu erreichen, bieten sich insbesondere Wirkstoffträger geeigneter Größen aus bioabbaubaren Polymeren an, wobei unter bioabbaubaren Polymeren im Sinne der vorliegenden Erfindung, derartige Polymere zu verstehen sind, die auf der Lungenoberfläche oder im menschlichen bzw. tierischen Körper zu pharmakologisch unbedenklichen Substanzen abgebaut werden.

Voraussetzung für eine unbedenkliche Anwendbarkeit derartiger inhalativer Wirkstoffträger, ist:
a) daß die Wirkstofffreigabe innerhalb des medizinisch angezeigten Zeitraums erfolgt;
b) daß das bioabbaubare Material des Wirkstoffträgers innerhalb eines - vom medizinischen Standpunkt - vertretbaren Zeitraums vom Organismus zu pharmakologisch unbedenklichen Produkten abgebaut wird;
c) daß die Materialeigenschaften der in Betracht kommenden bioabbaubaren Polymere es ermöglichen, Wirkstoffträger einer Größe herzustellen (Partikel-Durchmesser zwischen ca. 1 und 10 µm), die einen Einsatz in Form eines Aerosols ermöglichen;
Aus dem Stand der Technik sind einige Vorschläge für inhalative Depotformen auf der Basis bioabbaubarer polymerer Wirkstoffträger bekannt.

So offenbart die EP 257 915 eine auf Mikrokapseln basierende Depotform, die unter anderem für die inhalative Anwendung geeignet ist.

Desgleichen offenbart die EP 269 921 Mikrokapseln mit einem Durchmesser im Bereich von 0,1 bis 10 µm, deren polymeres Trägermaterial Polylactid ist und welche mittels Ultraschall erzeugt werden.

Weitere Verfahren zur Herstellung von Mikrokapseln in diesem Größenbereich sind aus den DE-OSen 23 60 384 und 26 11 143 sowie aus der Belgischen Patentschrift 869 107 bekannt. Bei diesen Verfahren werden Partikel mit einem Durchmesser von 20 bis 500 nm erhalten.

Die Herstellung inhalativer Wirkstoffträger auf der Basis von mikroverkapselten Systemen weist jedoch den Nachteil auf, daß die Herstellung derartiger Mikrokapseln, trotz zahlreicher Verbesserungen immer noch vergleichsweise aufwendig ist.

Bei der Verwendung von mikroverkapselten Systemen als Träger von Arzneistoffen ergeben sich besonders im Hinblick auf das Herstellungsverfahren folgende Nachteile:
1. Es muß ein Lösungsmittelpaar gefunden werden, das eine Emulsionsbildung ermöglicht, wobei beide Lösungsmittel mit dem Wirkstoff verträglich sein müssen;
2. Das Lösungsmittel, welches die äußere Phase in der Emulsion bildet, muß zudem derart beschaffen sein, daß es möglichst wenig von dem Wirkstoff löst, der sonst den Mikrokapseln verloren ginge;
3. Es müssen in zahlreichen Fällen oberflächenaktive Stoffe als Stabilisatoren bei der Mikroverkapselung eingesetzt werden, welche selbst pharmazeutisch unbedenklich und gegenüber dem Wirkstoff inert sein müssen;
4. Die Verwendung von Ultraschall bei der Herstellung von Mikrokapseln kann bei einer längeren Anwendung der Beschallung zu einer chemischen Veränderung (Sonochemie/lokale thermische Belastung) des Arzneistoffes oder des eingesetzten Polymeren bzw. anderer Inhaltsstoffe der Emulsion führen. Ferner stehen, bedingt durch den beschränkten Einsatz, den die Verwendung von Ultraschall in der Chemie bislang erfahren hat, hauptsächlich nur Apparaturen für die Anwendung im Labormaßstab zur Verfügung.

Der Einsatz der mit Ultraschall arbeitenden Verfahren erscheint somit - besonders unter dem Blickwinkel einer Herstellung in industriellen Größenordnungen - als unvorteilhaft.

Ferner besteht beim Entfernen der bzw. des Lösungsmittel(s) im letzten Herstellungsschritt bei der Anwendung der Sprühtrocknung - welche ein Standardverfahren bei der Herstellung von Mikrokapseln darstellt - die Gefahr, daß auch hier der Wirkstoff eine thermische Schädigung erfährt.

Auch das in der DE-OS 33 41 001 offenbarte Verfahren vermag diese Nachteile nicht zu überwinden, zumal bei dessen Anwendung sog. "Nanopartikel" mit einer Teilchengröße kleiner 1 µm resultieren. Bei Partikeln dieser Größenordnung besteht die Gefahr, daß sie sich infolge des aus der Größe resultierenden geringen Gewichts - bei der Anwendung als Aerosol - nicht auf der Lungenoberfläche absetzen, sondern im Laufe relativ kurzer Zeit als Schwebstoffe wieder ausgeatmet werden, ohne ihren Wirkstoff abgeben zu können [D. Köhler, W. Fleischer und H. Matthys, Inhaltionstherapie, Verlag Gedon und Reuss, München 1986, S. 9 und zit. Lit. - z.B.: T.T. Mercer Production of Therapeutic Aerosols-Principles and Techniques, Chest 80 (1981) 813].

Weiterhin besitzen derartige Polymere den Nachteil, daß sie in Abhängigkeit von dem jeweiligem Herstellungsverfahren Rückstände der bei der Polymerisation eingesetzten Katalysatoren aufweisen. So werden z.B. in industriellen Verfahren bei der ringöffnenden Polymerisation von cyclischen Estern üblicherweise Metallsalze bzw. metallorganische Verbindungen als Katalysatoren eingesetzt. die - wenn auch z.T. nur in geringen Mengen - im Polymerisat noch nachweisbar sind. Daraus erwächst bei einer längeren Anwendung auf der Basis derartiger Polymere hergestellter Arzneiformen die Gefahr, daß es mit der Zeit zu einer Akkumulation dieser Katalysatorrückstände in einzelnen Organen bzw. im ganzen menschlichen oder tierischen Körper kommt.

Die eingangs erwähnte Belgische Patentschrift ermöglicht zudem nur die Adsorption von Wirkstoffen, was zur Folge hat, daß die Beladung der einzelnen Partikel lediglich im Rahmen von 0,1 bis 1,5 Gewichtsprozent möglich ist und daher ein therapeutischer Effekt nur durch Verabreichung einer unverhältnismäßig großen Menge an Partikeln zu erreichen ist [Brasseur et al., European Journal of Cancer 16 (1980) 1441; Couvreur et al., Journal of Pharmacy and Pharmacology 31 (1979) 331; Abdel El Egakey et al., Pharmaceutica Acta Helvetica 57 (8), (1982) 236].

Des weiteren eröffnet dieses Verfahren keine Möglichkeit, Arzneistoffe zu verkapseln, die in Wasser nur wenig löslich sind, da die Polymerisation in wässerigem Medium durchgeführt werden muß, welches den Wirkstoff in geringer Konzentration aufweisen muß.

Es ist die Aufgabe der vorliegenden Erfindung, ein Verfahren zur Herstellung der erfindungsgemäßen mikronisierten wirkstoffhaltigen Partikel vorzuschlagen, bei dem ein mit Wirkstoff beladenes Trägermaterial - ohne thermische Belastung und/oder Ultraschallbelastung auf eine Teilchengröße gebracht wird, welche den Einsatz dieser Wirkstoff tragenden Partikel als Depotaerosol ermöglicht.

Daneben besteht eine weiteres Ziel der vorliegenden Erfindung darin, mikronisierte Partikel aus einem Material zur Verfügung zu stellen, welches frei von Katalysatorrückständen ist.

Die Aufgabe der vorliegenden Erfindung wird erfindungsgemäß durch ein Verfahren gelöst, bei dem wirkstoffbeladene bioabbaubare Polymere mittels Strahlenmahlung auf einen Korngrößendurchmesser im Bereich von 1 bis 10 µm gemahlen werden.

Als bioabbaubare Polymere eignen sich alle - ggf. katalysatorfreien - Polymere, welche entsprechende kristalline Eigenschaften bzw. eine ausreichende Härte aufweisen, die ihrerseits eine Strahlmahlung ermöglicht. Bevorzugt werden Polylactide mit einem Molekulargewicht von 1000 bis 10 000, wobei Poly-L(-)-lactid mit einem Molekulargewicht im Bereich von 1000 bis 5000 besonders bevorzugt wird.

Das Molekulargewicht repräsentiert einen Parameter, über den sich der Diffusionskoeffizient und damit die Freigabegeschwindigkeit des Arzneistoffes steuern läßt. Weitere Parameter sind zum einen die Korngrößenverteilung der mit Arzneistoff beladenen Partikel und zum anderen der Beladungsgrad Arzneistoff/Polymer (siehe Tabelle 3).

Die Herstellung eines katalysatorfreien Poly-L(-)-lactids, dessen Molekulargewicht in den angegebenen Bereichen liegt, ist aus der europäischen Patentschrift 261 572 bekannt.

Es ist jedoch auch der Einsatz anderer bioabbaubarer Polymere oder Copolymere denkbar, soweit diese für das Strahlmahlverfahren geeignete kristalline Eigenschaften aufweisen. Als Beispiele seien genannt: Homo- und Copolymere auf der Basis von Hydroxycarbonsäuren, wie beispielsweise Polymerisate von Glycolid, Lactid, Methylglycolid, Dimethylglycolid, Polymethylglycolid, Diethylglycolid, Dibutylglycolid, Caprolacton, Valerolacton, Decalacton, Propiolacton, Butyrolacton, Pivalolacton, sowie Polymere auf der Basis von Trioxanon, Dioxanon (1,3 und 1,4), substituiertem Dioxanon, Trimethylencarbonat, Ethylencarbonat und Propylencarbonat.

Als weitere Comonomere sind u.a. die folgenden Verbindungen geeignet:
Milchsäure, Glykolsäure, Pentaerythrit, Sorbit, Adonit, Xylit, Fructose, Epichlorhydrin, Isopropylmorpholin, Isopropylmethylmorpholindion, β-Propiolsäure, Tetramethylglycolid, β-Butyrolacton, γ-Butyrolacton, Pivalolacton, α-Hydroxybuttersäure, α-Hydroxyisobuttersäure, α-Hydroxyvaleriansäure, α-Hydroxyisovaleriansäure, α-Hydroxycapronsäure, α-Hydroxyisocapronsäure, α-Hydroxy-α-ethylbuttersaure, α-Hydroxy-α-methylvaleriansäure, α-Hydroxyheptansäure, α-Hydroxyoctansäure, α-Hydroxydecansäure, α-Hydroxytetradecansäure, α-Hydroxystearinsäure.

Als Arzneistoffe eignen sich alle diejenigen Arzneistoffe, bei denen eine Anwendung in Form eines Aerosols sinnvoll erscheint - beispielsweise Anticholinergica, Antiallergica, Steroide, β-Sympathomimetica. Als Beispiele seien genannt:
(a) 1-(3,5-Dihydroxylphenyl)-1-hydroxy-2-[(4-hydroxyphenyl)isopropylamino]ethan (Fenoterol)
(b) 4-Amino-α-[tert.-butylamino)methyl]-3,5-dichchlorbenzylalkohol (Clenbuterol)
(c) 2-Hydroxymethyl-3-hydroxy-6-(1-hydroxy-2-tert-butylaminoethyl)pyridin (Pirbuterol)
(d) 8-Hydroxy-5-[1-hydroxy-2-[(1-methylethyl)amino]butyl]-2(1H)-chinolinon (Procaterol)
(e) 2-(tert.-Butylamino)-1-(4-hydroxy-3-hydroxymethylphenyl)ethanol (Salbutamol)
(f) 1-(3,5-Dihydroxyphenyl)-2-(tert.-butylamino-) ethanol (Terbutalin)
(g) 1-(2-Fluor-4-hydroxylphenyl)-2-[4-(1-benzimidazolyl)-2-methyl-2-butylamino]-ethanol
(h) erythro-5′-Hydroxy-8′-(1-hydroxy-2-isopropylaminobutyl)-2H-1,4-benzoxazin-3(4H)-on
(i) 1-(4-Amino-3-chlor-5-trifluormethylphenyl)-2-(tert.-butylamino)ethanol
(k) 1-(4-Ethoxycarbonylamino-3-cyan-5-fluorphenyl)-2-(tert.-butylamino)ethanol
(l) N,N′-Bis[2-(3,4-dihydroxyphenyl)-2-hydroxyethyl]hexamethylendiamin (Hexoprenalin)
(m) 7-[3-[[2-(3,5-Dihydroxyphenyl)-2-hydroxyethyl-] amino]propyl]-3,7-dihydro-1,3-dimethyl-1H-purin-2,6-dion (Reproterol)
(n)-[5-[2-[(1,1-Dimethylethyl)amino]-1-hydroxyethyl]-2-hydroxyphenyl]-harnstoff (Carbuterol)
(o) 2-Chlor-α-[[(1,1-dimethylethyl)-amino]-methyl-] benzylalkohol (Tulobuterol)
(p) 3-Formylamino-4-hydroxy-α[N-[1-methyl-2-p-methoxyphenyl)ethyl]-aminomethyl]benzyalkohol Semifumarat (Formoterol)
(q) N-[2-Hydroxy-5-[1-hydroxy-2-[[2-(4-methoxyphenyl)-1-methylethyl]amino]ethyl]phenyl]-formamid Semifumarat
(r) α[(tert.Butylamino)-methyl-4-hydroxy-3(methylsulfonyl)methyl]benzylalkohol Hydrochlorid (Sulfonterol)
(s) 5-[2-(tert.-Butylamino)-1-hydroxyethyl]-m-phenylenbisdimethylcarbamat) (Bambuterol)
(t) 5-[2-(tert.-Butylamino)-1-hydroxyethyl]-m-phenylendiisobutyrat (Ibuterol)
(u) 4-[2-(tert. Butylamino)-1-hydroxyethyl]-1,2-phenylen-di-4-toluat (Bitolterol)
(v) 4-Hydroxy-α[[(6-(4-phenylbutoxy]hexylamino]methyl-m-xylen-α,α′diol (Salmeterol)
(w) N,N-Dimethyl-N-(3-sulfopropyl)-4-[2[2-(2,3-dihydro-5-hydroxy-3-oxo-4H-1,4-benzoxazin-8-yl]-2,2-dimethylethyl]phenoxyamonium-hydroxid-hydrochloridmonohydrat . H₂O [inneres Salz]
(x) 5,5′-(2-Hydroxy-trimethylendioxy)-bis(4-oxochromen-2-carbonsäure (Cromoglycinsäure)
(y) 9-Ethyl-6,9-dihydro-4,6-dioxo-10-propyl-4H-pyrano[3,2-g]chinolin-2,8-dicarbonsäure (Nedocromil)
Die Beladung erfolgt zweckmäßigerweise in der Art, daß das bioabbaubare Polymer und der Arzneistoff in jeweils geeigneten Lösungsmitteln gelöst werden.

Als Beispiele seien genannt: Ether - wie Glykoldimethylether, Tetrahydrofuran und Dioxan - Ketone - wie z.B. Aceton - Amide - wie z.B. Dimethylformamid - oder chlorierte Kohlenwasserstoffe - wie z.B. Dichlormethan oder Chlorform oder Lösungsmittelgemische.

Als Lösungsmittel für das bioabbaubare Polymer bzw. Copolymer eignen sich die aus dem Stand der Technik bekannten Lösungsmittel, soweit sie sich dem Arzneistoff gegenüber inert verhalten.

Für Polylactide eignen sich halogenierte Kohlenwasserstoffe wobei chlorierte Kohlenwasserstoffe bevorzugt werden; für Poly-L(-)-lactid wird Dichlormethan besonders bevorzugt.

Für den Arzneistoff eignen sich alle inerten Lösungsmittel, die weder mit dem Arzneistoff noch mit dem bioabbaubaren Polymer in nachteiliger Weise reagieren und die problemlos aus der Arzneimittelzubereitung wieder zu entfernen sind. Als Beispiele seien halogenierte Kohlenwasserstoffe - wie beispielsweise Chloroform, Methylenchlorid (Dichlormethan) - oder Ketone - wie z.B. Aceton oder Ethylmethylketon oder Ether - wie z.B. Glykoldimethylether oder Tetrahydrofuran genannt. Selbstverständlich können als Lösungsmittel sowohl für das bioabbaubare Polymer als auch für den Arzneistoff Lösungsmittelmischungen eingesetzt werden.

Es ist zudem möglich, weitere Hilfsstoffe in die Lösung des bioabbaubaren Polymeren oder in die Lösung des Arzneistoffs mit aufzunehmen.

Als Hilfsstoffe finden dabei insbesondere Antistatika - wie z.B. Soja - Lecitin Verwendung, um den Mahlprozess zu erleichtern und um eine elektrostatische Agglomeration der Partikel zu verhindern.

Als weitere Hilfsstoffe können gegebenenfalls geruchsmaskierende Substanzen dienen, die - erforderlichenfalls - den Eigengeruch des Arzneimittels kaschieren oder pharmakologisch unbedenkliche Stabilisatoren oder Konservierungsstoffe.

Die Herstellung der mikronisierten bioabbaubaren Partikel erfolgt erfindungsgemäß in der Weise, daß zunächst eine Lösung des bioabbaubaren Polymeren - mit gegebenenfalls einzusetzenden Hilfsstoffen - und eine Lösung des Arzneistoffes, die gewünschtenfalls auch noch (weitere) Hilfsstoffe aufweisen kann, vereinigt werden.

Es ist jedoch auch möglich - bei Verwendung eines gemeinsamen Lösungsmittels für Polymer und Arzneistoff - alle Bestandteile von vornherein in einer Lösung zu vereinigen.

Die resultierende Mischung wird vom Lösungsmittel befreit - was in Abhängigkeit von den Siedepunkten der eingesetzten Lösungsmittel und der thermischen Belastbarkeit des Arzneistoffes gegebenenfalls unter vermindertem Druck erfolgen kann.

Der resultierende Feststoff wird nach dem Trocknen erforderlichenfalls vorzerkleinert und dann der Strahlmahlung unterworfen, woraus Partikel, die einen mittleren Durchmesser im Größenbereich von 1 bis 10 µm aufweisen. Die Strahlmahlung weist den weiteren Vorteil auf, daß die Temperatur, bei der der Mahlvorgang erfolgt in weiten Bereichen regelbar ist, was im Hinblick auf das Mahlgut den Vorzug mit sich bringt, daß Polymere, deren kristalline Eigenschaften bzw. Härte eine Strahlmahlung nicht erlaubt gegebenenfalls bei tieferen Temperaturen zu Partikeln der gewünschten Teilchengröße zerkleinert werden können. Das Verfahren der Strahlmahlung ist aus dem Stand der Technik bekannt [Ullmann's Encyclopedia of Industrial Chemistry, Vol. B2: Unit Operations I, VCH Verlagsgesellschaft mbH, D-6940 Weinheim, Seite 5-30 ff].

Aus dem erfindungsgemäßen Herstellungsverfahren resultieren mikronisierte bioabbaubare Partikel in Form eines inhalierbaren Pulvers, das als Depotaerosol - auch ohne die Anwendung von Treibgas erforderlich zu machen - eingesetzt werden können (Beispielsweise kann das Pulver in eine Hartgelatine-Kapsel abgefüllt werden unu von Patienten mittels einer geeigneten Inhalationsvorrichtung inhaliert werden).

Die nach dem erfindungsgemäßen Verfahren hergestellten bioabbaubaren Partikel können auch in Form von Injektionslösungen Verwendung finden - insbesondere in Zubereitungen, die für die intramuskuläre oder intraartikuläre Injektion bestimmt sind. Der besondere Vorteil, den die Verwendung der erfindungsgemäßen katalysatorfreien Partikel z.B. bei der intraartikulären Injektion mit sich bringt, besteht darin, daß der Arzneistoff in der gewünschten Zielregion freigesetzt werden kann, ohne daß es dabei durch das abgelagerte Polymer oder - bei einer längeren Behandlungsdauer - durch akkumulierte Katalysatorreste zu einer lokalen Irritation des umliegenden Gewebes kommt.

Die nach dem erfindungsgemäßen Verfahren hergestellten bioabbaubaren Partikel eignen sich ebenso zur oralen Applikation - beispielsweise in Form einer Kapsel. Auch bei dieser Applikationsform ist mit einer gleichmäßigen Wirkstoffabgabe über einen längeren Zeitraum zu rechnen, da anzunehmen ist, daß sich der Kapselinhalt sehr gut im Intestinum verteilt (Das Mischungsverhalten der mikronisierten Partikel mit dem Speisebrei dürfte in erster Näherung mit demjenigen einer Flüssigkeit gleichzusetzen sein).

Hinsichtlich der verwendbaren Arzneistoffe lassen sich die eingangs genannten Pharmazeutika in Abhängigkeit von der jeweiligen Indikation durch Arzneistoffe - wie beispielsweise Antiphlogistika oder Cytostatika - entsprechend ergänzen.

Die mit Poly-L(-)-lactid als bioabbaubaren Polymer und Fenoterol als Arzneistoff hergestellten mikronisierten Partikel zeigen im in vitro und im in vivo Freigabeverhalten, daß mit den erfindungsgemäßen Partikeln eine kontrollierte Freisetzung über einen mehrstündigen Zeitraum erzielt werden kann.

### Anwendungsbeispiel

Das nachfolgende Beispiel erläutert die Erfindung ohne sie einzuschränken:
2.1. Für die Untersuchungen wurde als Arneistoff Fenoterol-Base und als bioabbaubares Polymeres Poly-L(-)-Milchsäure vom Molekulargewicht 2000 eingesetzt.
Als weiterer Hilfsstoff fand Soja-Lecithin als Antistatikum Verwendung. Als organische Lösungsmittel dienten Methylenchlorid und Aceton p.a.
Die quantitativen Angaben für ein typisches Beispiel sind in Tabelle 1 zusammengestellt.

**Tabelle 1**

| Menge | Substanzbezeichnung |
|---|---|
| 50 g | Poly-L(-)-Milchsäure |
| 2,50 g | Fenoterol-Base |
| 1,5 g | Soja-Lecithin |
| 200 g | Methylenchlorid |
| 100 g | Aceton |

Die Herstellung erfolgte in der Weise, daß die Poly-L(-)-Milchsäure und das Soja-Lecithin in Methylenchlorid und in einem weiteren Gefäß die Fenoterol-Base in Aceton aufgelöst wurden. Beide Lösungen wurden dann zusammengefügt, vom Lösungsmittel befreit und anschließend auf einer Horde bei 40°C im Trockenschrank 18 h lang getrocknet. Nach dem Trocknungsprozeß resultierte ein weißes Material, das zunächst mit einem Mörser vorzerkleinert und anschließend mittels Strahlmahlung (Mahldauer 3 Min., Mahlluft 5,5 atü, Speiseluft 5,0 atü) in ein mikronisiertes Pulver überführt wird (2˝-microniser, Sturtevent Mill Company Dorchester, Boston 22, Massachusetts). Die quantitative Korngrößenanalyse mittels eines handelsüblichen Teilchengrößenanalysators ergab, daß der mittlere Durchmesser d_{3/50} bei 2.4 µm lag.

**Tabelle 2**

| Korngrößenverteilung des strahlengemahlenen Polylactid/Fenoterol-Pulvers (Meßmethode HIAC) | |
|---|---|
| Durchmesser in µm | Prozentualer Volumenanteil |
| 1.1 | 0.63 |
| 1.3 | 6.83 |
| 1.8 | 25.13 |
| 2.1 | 38.59 |
| 2.5 | 55.70 |
| 2.9 | 68.01 |
| 3.5 | 80.54 |
| 4.8 | 89.37 |
| 6.7 | 93.55 |
| 9.4 | 95.15 |

Die Freigabe des Fenoterols aus dem Polymerträger wurde im Paddle-Tester (USP XXI) in 0,05 % wässeriger Benzalkoniumchloridlösung (T = 21°C, Rührgeschwindigkeit 200 Umdrehungen pro Minute) bestimmt. Diese in vitro Prozeßparameter wurden so festgelegt, daß eine Differenzierung in der Freigaberate zwischen Versuchsformulerierungen möglich war. Die Bestimmung an Fenoterol erfolgte mittels der HPLC-Methodik.
Für die pharmakologischen Inhalationsexperimente wurde die jeweilige Menge an mikroniersiertem Pulver (z.B. für 200 µg Fenoterol HBr = 4,32 mg Gesamtmenge) abgewogen und in Hartgelatinekapseln überführt. Die Desagglomeration und damit Zuführung des Pulvers im Tierversuch erfolgte mittels einer Inhalationsapparatur, wobei das erzeugte Aerosol im Tierexperiment direkt in die Trachea gelenkt wurde (Methode nach Konzett und Rössler, modifiziert).
2.2. Freigabemengen in % einer Formulierung mit einem Beladungsgrad (Wirkstoff/Polymer) von 4,6 % und 2,4 %

**Tabelle 3**

| Freigabemengen in % | | |
|---|---|---|
| Freigabe Zeit in Min. | 4,6 % Beladungsgrad | 2,4 % Beladungsgrad |
| 15 min. | 54,2 % | 44,4 % |
| 60 min. | 65,7 % | 51,8 % |
| 120 min. | 70,9 % | 59,6 % |
| 240 min. | 75,7 % | 62,7 % |

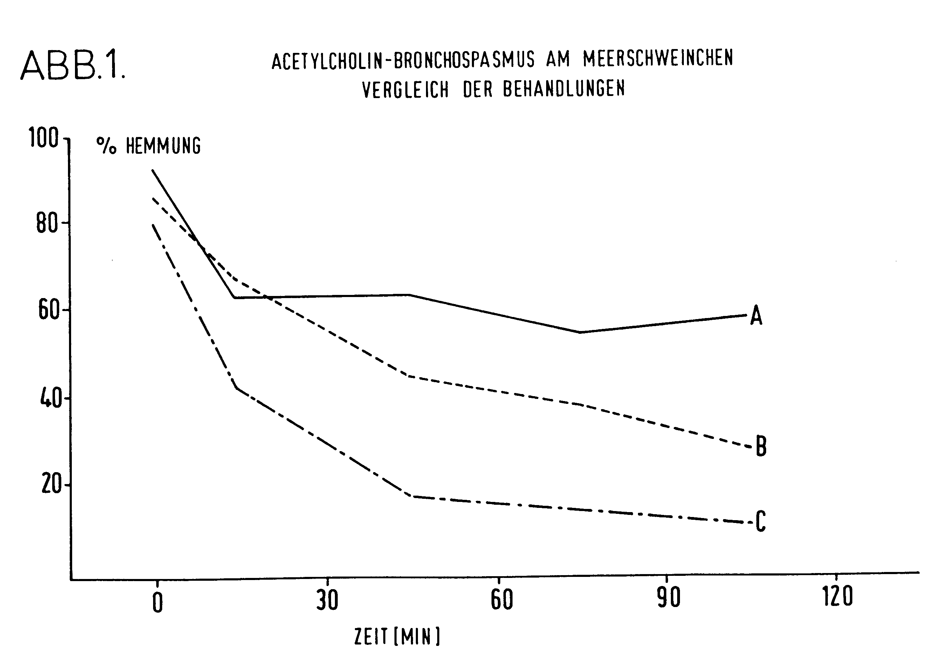

2.3 Tierexperimentelle Methodik für die Erfassung der Bronchospasmolytischen Wirkung am narkotisierten Meerschweinchen
Die Methode von Gjurisch et al. (F. Gjurisch, B. Heike und E. Westermann, Naunyn-Schmiedeberg's Arch. exp. Pathol. Pharmakol. 247 (1964), 429 wurde modifiziert. Für die Versuche dienten weibliche Albino-Meerschweinchen (Pirbright White, Ivanovas, gefüttert mit Sniff, Körpergewicht 300-400 g).
Die Tiere wurden mit 1,8 g/kg Ethylurethan (als 25 %ige wässerige Lösung) i.p. anästhesiert. Anschließend erfolgte die Tracheotomie und die Kanülierung der Vena jugularis. Muskelrelaxation mit 3 mg/kg Flaxedil i.v. Die Meerschweinchen wurden an eine Atempumpe für Kleintiere angeschlossen: Hubvolumen 5 ml, Frequenz 60 Hübe/min., Beatmungsdruck konstant 10 ml Wassersäule durch ein im Seitenanschluß befindlichen Wasserüberdruckventil (Konzett und Rössler). Die Körpertemperatur wurde auf etwa 38°C konstant gehalten (Heizung mit Temperaturfühler, der mit einem elektronischen Temperaturregler verbunden war). Das Atemvolumen wurde in einem Körperlethysmografen (Durchmesser 9,7 cm, Länge 28,5 cm, lichte Höhe 8,3 cm, luftdichter Abschluß mittels Glasplatte, Anschlüsse für EKG-Elektroden und einen Katheter zur i.v. Injektion) gemessen. Für die inhalative Verabreichung wurde je 1 Hub des Aerosols (Depotformulierung oder Fenoterol-Glukose-Aerosol) direkt in die Trachea verabreicht. Während der Applikation wurde die Verbindung zwischen der Trachea (bzw. Trachealschlauch) und Fleisch'schem Staurohr (0000, Größe für Kleintiere) kurz unterbrochen. Die Änderungen des Atemdruckes wurden mittels eines elektronischen Druckwandlers erfaßt und eines Schreibers registriert. Nach einer 30-minütigen Eingewöhnungspause wurde mit dem Versuch begonnen. Zur Auslösung der Bronchospasmen wurden 35-50 µg/kg Acetylcholin (im Abstand von 10 Minuten) intravenös injiziert, bis gleichmäßige Bronchospasmen (mit den letzten zwei Applikationen) feststellbar waren. Danach wurden die Prüfformulierungen inhalatorisch verabreicht und die Spasmogene in Abständen von 10 Minuten intravenös injiziert.
Die Versuchsergebnisse sind in Abbildung 1 widergegeben:
Abbildung 1 zeigt die prozentuale Hemmung von durch Acetylcholin induzierte Bronchospasmen als Funktion der Zeit.
- Kurve A:: Beladungsgrad 2,4 %
- Kurve B:: Beladungsgrad 4,6 %
- Kurve C:: Fenoterol-Glukose-Aerosol
Wie die Biometrie zeigt, sind die maximalen Effekte zwischen den Poly-L(-)-Milchsäure-Depotaerosolen mit 2,4 % bzw. 4,6 % Beladung (Kurve A bzw. B) gegenüber einem Fenoterol-Glukose-Aerosol (Kurve C) in den ersten 15 Minuten vergleichbar - nach diesem Zeitraum zeigen die Funktionen jedoch einen signifikant unterschiedlichen Verlauf wobei die Zubereitung mit 2,4 % Fenoterol hinsichtlich der verzögerten Freigabe die besten Ergebnisse lieferte.
2.4 Weitere Anwendungsbeispiele
In Analogie zu dem vorstehend beschriebenen Verfahren wurden Arzneimittelzubereitungen mit Salbutamol-Base und Ipratropium-Bromid hergestellt. Die Trocknung, Aufarbeitung und Mahlung des Materials erfolgte dabei unter den gleichen Bedingungen wie vorstehend für die Zubereitung mit der Fenoterol-Base beschrieben.
Die Korngrößenverteilung, die wiederum mit der HIAC-Meßmethode bestimmt wurde, liegt in dem gleichen Bereich wie für das System Fenoterol/Poly-Milchsäure beschrieben.
Die Bestimmung der Freigaberaten des jeweiligen Wirkstoffes erfolgte ebenfalls unter den gleichen Bedingungen wie für Fenoterol-Base zuvor beschrieben. Für die analytische Bestimmung wurde auf die HPLC-Methode zurückgegriffen:
2.4.1. Salbutamol-Zubereitung
Zusammensetzung:
50 g Poly-L-Milchsäure
2,5 g (1,25 g) Salbutamol
1,5 g Soja-Lecitin
200 g Dichlormethan
100 g Ethanol

| Freigabemenge in %: | | |
|---|---|---|
| Freigabezeit in Minuten | 4,6 % Beladung | 2,4 % Beladung |
| 15 | 63.3 | 52.3 |
| 60 | 71.1 | 56.0 |
| 120 | 74.2 | 60.3 |
| 240 | 75.3 | 69.9 |

2.4.2. Ipratropium-Zubereitung
Zusammensetzung
50 g Poly-L-Milchsäure
2,5 g Ipratropium-Bromid
1,5 g Soja-Lecitin
200 g Dichlormethan
100 g Ethanol

| Freigabemenge in %: | |
|---|---|
| Freigabezeit in Minuten | 4,6 % Beladung |
| 15 | 53.6 |
| 60 | 58.0 |
| 120 | 61.1 |
| 240 | 67.0 |

## Patentansprüche

1. Verfahren zur Herstellung mikronisierter Teilchen, dadurch gekennzeichnet, daß man wirkstoffbeladene bioabbaubare polymere Teilchen mittels Strahlenmahlung auf einen Korngrößendurchmesser von 1 bis 10 µm mahlt.

2. Verfahren nach Anspruch 1, dadurch gekennzeichnet, daß das bioabbaubare Polymer Poly-L-(-)-lactid ist.

3. Verfahren zur Herstellung mikronisierter bioabbaubarer Partikel gemäß einem der Ansprüche 1 oder 2, dadurch gekennzeichnet, daß man als bioabbaubares Polymer ein Poly-L-(-)-lactid mit einem Molekulargewicht im Bereich von 1000 bis 10 000 einsetzt.

4. Verfahren zur Herstellung mikronisierter Partikel gemäß einem der Ansprüche 1 bis 3, dadurch gekennzeichnet, daß man als pharmazeutischen Wirkstoff einen Wirkstoff aus der Gruppe der Anticholinergica, Antiallergica, Steroide und/oder der β-Sympathomimetica einsetzt.

5. Verfahren zur Herstellung mikronisierter Partikel gemäß einem der Ansprüche 1 oder 2, dadurch gekennzeichnet, daß man als bioabbaubares Polymer ein Poly-L(-)-lactid mit einem Molekulargewicht im Bereich von 1000 bis 3000 und als pharmazeutischen Wirkstoff Fenoterol einsetzt.

## Claims

1. Process for preparing micronised particles, characterised in that biodegradable polymer particles charged with active substance are ground to a particle size in the range from 1 to 10 µm by jet grinding.

2. Process according to claim 1, characterised in that the biodegradable polymer is poly-L(-)-lactide.

3. Process for preparing micronised biodegradable particles according to one of claims 1 or 2, characterised in that the biodegradable polymer used is a poly-L(-)-lactide having a molecular weight in the range from 1000 to 10,000.

4. Process for preparing micronised particles according to one of claims 1 to 3, characterised in that the pharmaceutical active substance used is an active substance from the groups comprising the anticholinergics, antiallergics, steroids and/or β-sympathomimetics.

5. Process for preparing micronised particles according to one of claims 1 or 2, characterised in that the biodegradable polymer used is a poly-L(-)-lactide having a molecular weight in the range from 1000 to 3000 and the pharmaceutical active substance used is fenoterol.

## Revendications

1. Procédé de préparation de particules micronisées, caractérisé en ce que l'on broie à un diamètre de grains de 1 à 10 µm, par broyage à jet, des particules polymériques biodégradables chargées de principe actif.

2. Procédé selon la revendication 1, caractérisé en ce que le polymère biodégradable est un poly-L-(-)-lactide.

3. Procédé de préparation de particules biodégradables micronisées selon l'une des revendications 1 ou 2, caractérisé en ce que l'on utilise comme polymère biodégradable un poly-L-(-)-lactide ayant une masse moléculaire dans le domaine de 1000 à 10000.

4. Procédé de préparation de particules micronisées selon l'une des revendications 1 à 3, caractérisé en ce que l'on utilise comme principe actif pharmaceutique un principe actif du groupe des anticholinergiques, des antiallergiques, des stéroïdes et/ou des β-sympathomimétiques.

5. Procédé de préparation de particules micronisées selon l'une des revendications 1 ou 2, caractérisé en ce que l'on utilise comme polymère biodégradable un poly-L-(-)-lactide ayant une masse moléculaire dans le domaine de 1000 à 3000 et comme principe actif pharmaceutique le fénotérol.
